# EUROPEAN PATENT APPLICATION

(11) **EP 1 504 756 A1**
(43) Date of publication of application: **09.02.2005**
(21) Application number: 03017943.6
(22) Date of filing: 06.08.2003
(51) Int. Cl.: A61K 31/46, A61K 31/357, A61P 11/00, A61P 11/08

(54) **Medicament compositions comprising a heterocyclic compound and an anticholinergic**

(71) Applicant: Kyowa Hakko Kogyo Co., Ltd, Tokyo 100-8185 (JP)
(72) Inventor: Meade, Christopher J. Montague, Dr., 88437 Maselheim (DE); Pairet, Michel, Dr., 88400 Biberach (DE); Pieper, Michael P., Dr., 88400 Biberach (DE); Abe, Yuzuru, 411-8731 Shizuoka (JP); Miki, Ichiro, Shizuoka, 411-8731 (JP)
(74) Representative: VOSSIUS & PARTNER

(57) **Abstract**

The present invention relates to novel pharmaceutical compositions based on anticholinergics of the formula **1** 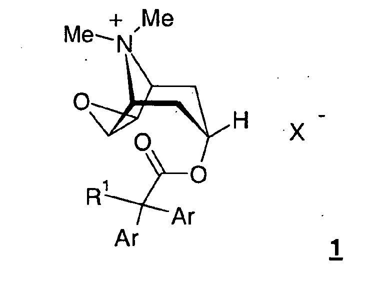 wherein X⁻, R¹ and Ar are defined as in claim 1, and a heterocyclic compound of the formula **2** 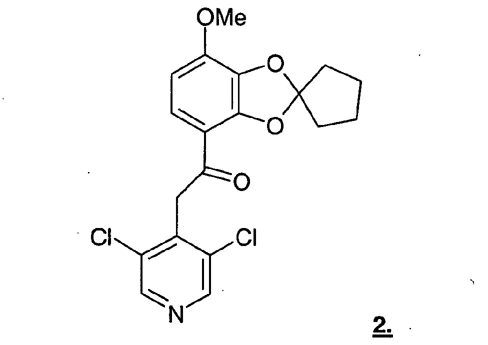 Furthermore, the invention relates to processes for preparing them and their use in the treatment of respiratory complaints.

## Description

The present invention relates to novel pharmaceutical compositions based on anticholinergics of the formula **1** and a heterocyclic compound of the formula **2,** both as defined hereinafter, and to the use of such a composition. Furthermore the present invention relates to capsules containing an inhalable powder and to the use of such capsules. In addition, this invention relates to the use of an inhalable solution. The present invention also relates to a method of prophylaxis of, treating of, or reducing the exacerbations associated with, a pulmonary disease in a patient in need thereof. Furthermore the invention relates to a package comprising a pharmaceutical composition and to an inhaler.

The compound of the formula **2** and its manufacture are known from WO 96/36624. The compounds described therein exhibit phosphodiesterase (PDE) IV inhibitory activity.

A combination of PDE IV inhibitors and tiotropium for treating obstructive airways and other inflammatory diseases is described in the WO 02/096423.

Furthermore, a method of treating pulmonary diseases such as obstructive disease or asthma by administering a PDE IV inhibitor in combination with an anticholinergic agent is described in the WO 03/011274.

### Summary of the invention

The present invention relates to novel pharmaceutical compositions based on anticholinergics of the formula **1** 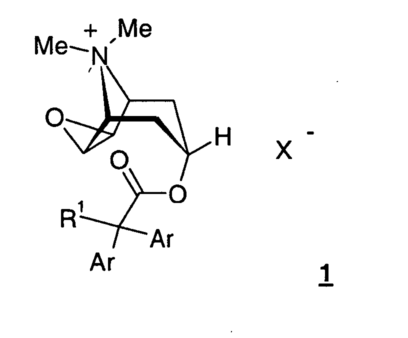 wherein
- X⁻: represents chlorine, bromine, iodine, methanesulphonate or trifluoromethanesulphonate;
- R¹: represents hydroxy or methyl;
- Ar: represents phenyl or thienyl, preferably 2-thienyl;
and a heterocyclic compound of the formula **2** 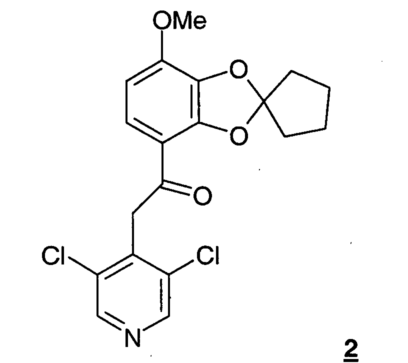 optionally in the form of a pharmacologically acceptable acid addition salt thereof, optionally in the form of a solvate or hydrate and optionally together with a pharmaceutically acceptable excipient.

The present invention further relates to capsules characterised in that they contain an inhalable powder which contains **1** and **2** in admixture with suitable physiologically acceptable excipients selected from among the monosaccharides, disaccharides, oligo- and polysaccharides, polyalcohols, salts, or mixtures of these excipients with one another.

This invention also relates to the use of a capsule according to this invention in an inhaler.

Furthermore, this invention relates to the use of a pharmaceutical composition in the form of an inhalable solution according to this invention for nebulising in an inhaler, preferably an inhaler according to WO 91/1 4468 or an inhaler as described according to the Figures 6a and 6b of WO 97/12687.

In addition, this invention relates to the use of a pharmaceutical composition according to this invention for preparing a medicament for the prophylaxis of, treating of, or reducing the exacerbations associated with pulmonary diseases, in particular of inflammatory or obstructive diseases of the respiratory tract.

This invention also relates to the prophylaxis of, treating of, or reducing the exacerbations associated with pulmonary diseases, in particular inflammatory or obstructive diseases of the respiratory tract, by administering to a patient in need thereof an effective amount of a pharmaceutical composition according to this invention either in a single combined form, separately, or separately and sequentially where the sequential administration is close in time, or remote in time.

Furthermore, this invention relates to a package comprising a pharmaceutical composition according to this invention for insertion into a device of simultaneous or sequential delivery of said pharmaceutical composition in the form of an aerosol or dry powder dispersion, to a mammal in need of treatment.

The.present invention also relates to an inhaler comprising a pharmaceutical composition according to the invention for simultaneous or sequential delivery of said pharmaceutical composition in the form of an aerosol or dry powder dispersion, to a mammal in need of treatment.

### Detailed description of the invention

Surprisingly, an unexpectedly beneficial therapeutic effect can be observed in the treatment of inflammatory and/or obstructive diseases of the respiratory tract if an anticholinergic of the formula **1** is used with a compound of formula **2**. Among other beneficial effects, a synergistic effect can be observed. A synergistic effect may have the advantage that the pharmaceutical combinations according to the invention can be used in smaller doses than would be the case with the individual compounds used in monotherapy in the usual way.

The pharmaceutical composition of the present invention is useful in the treatment and/or prophylaxis of obstructive airways diseases, especially of asthma, chronic obstructive pulmonary disease (COPD) and other obstructive airways diseases exacerbated by heightened bronchial reflexes, inflammation, bronchial hyper-reactivity and bronchospasm, in particular COPD.

In particular, the combination of a compound of the formula **1** and a compound of the formula **2** are useful in the treatment of respiratory diseases and conditions comprising: asthma, acute respiratory distress syndrome, chronic pulmonary inflammatory disease, bronchitis, chronic bronchitis, chronic obstructive pulmonary (airway) disease, including the pulmonary symptoms associated with cystic fibrosis and mucoviscidosis; and silicosis; or immune diseases and conditions comprising: allergic rhinitis and chronic sinusitis.

The effects mentioned above may be observed both when the two active substances are administered simultaneously in a single active substance formulation and when they are administered successively in separate formulations. According to the invention, it is preferable to administer the two active substance ingredients simultaneously in a single formulation.

Accordingly, in one aspect, the present invention relates to a pharmaceutical composition, characterised in that it contains an anticholinergic **1** in combination with a compound of the formula **2**. The compound of the formula **2** may be present in the form of the pharmacologically acceptable acid addition salts thereof.

Any reference to the above compound **2** includes within the scope of the present invention a reference to any pharmacologically acceptable acid addition salts thereof which may exist. By the physiologically acceptable acid addition salts which may be formed from **2** are meant, for example, pharmaceutically acceptable salts selected from the salts of hydrochloric acid, hydrobromic acid, sulphuric acid, phosphoric acid, methanesulphonic acid, acetic acid, fumaric acid, succinic acid, lactic acid, citric acid, tartaric acid or maleic acid. Preferred salts of the compounds **2** according to the invention are those selected from the salts of hydrochloric acid, hydrobromic acid, sulphuric acid, phosphoric acid, methanesulphonic acid and acetic acid.

In the abovementioned salts of the anticholinergic of the formula **1** the corresponding cation represents the pharmacologically active ingredient.

Within the scope of the present patent application any reference to the above cations is indicated by the use of the term **1'.** Any reference to compounds **1** naturally also includes a reference to the components **1**' .

By the salts **1** which may be used within the scope of the present invention are meant the compounds which contain, in addition to the corresponding cation as counter-ion (anion), chloride, bromide, iodide, methanesulphonate or para-toluenesulphonate. Within the scope of the present invention, the methanesulphonate, chloride, bromide and iodide are preferred of all the salts **1**, the methanesulphonate and bromide being of particular importance. The most preferred meaning of X⁻ is a bromide anion.

According to a first preferred embodiment of this invention, the pharmaceutical composition comprises an anticholinergic of the formula **1a** 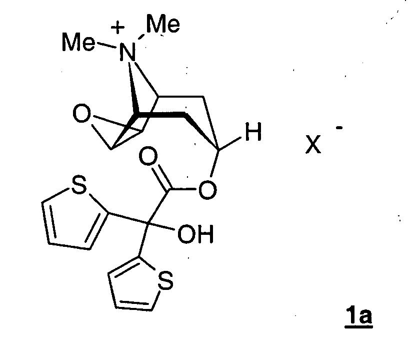 wherein
- X⁻: represents chlorine, bromine, iodine, methanesulphonate or trifluoromethanesulphonate.

Most preferably X⁻ is a bromide anion. Thus, the most preferred anticholinergic according to the first embodiment of this invention is tiotropium bromide, including tiotropium bromide monohydrate, preferably in form of its crystalline monohydrate as disclosed in WO 02/30928. In case tiotropium bromid is used in anhydrous form, it is preferably present in form of the crystalline tiotropium bromide anhydrate disclosed in WO 03/000265.

According to a second preferred embodiment of this invention, the pharmaceutical composition comprises an anticholinergic of the formula **1b** 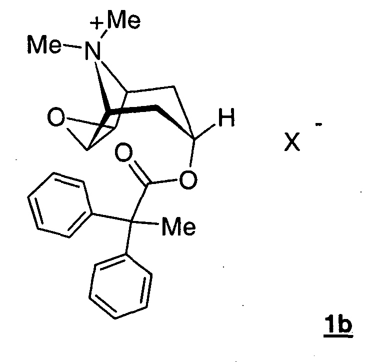 wherein
- X⁻: represents chlorine, bromine, iodine, methanesulphonate or trifluoromethanesulphonate.

Most preferably X⁻ is a bromide anion.

The compounds of formula **1b** are known in the art (WO 02/32899).

The pharmaceutical combinations of **1** and **2** according to the invention are preferably administered by inhalation. Suitable inhalable powders packed into suitable capsules (inhalettes) may be administered using suitable powder inhalers. Alternatively, the drug may be inhaled by the application of suitable inhalation aerosols. These also include inhalation aerosols which contain HFA134a (also known as TG134a), HFA227 (also known as TG227) or a mixture thereof as propellant gas. The drug may also be inhaled using suitable solutions of the pharmaceutical combination comprising of **1** and **2**. The single dose to be administered to the patient may be achieved by actuating the inhaler once, twice, three or more times, preferably by actuating the inhaler once or twice, most preferably once.

The active substances may be combined in a single preparation or contained in two separate formulations. Pharmaceutical compositions in which the active substances **1** and **2** are combined in a single preparation are preferred according to the invention.

In another aspect the present invention relates to a pharmaceutical composition which contains, in addition to therapeutically effective quantities of **1** and **2**, a pharmaceutically acceptable excipient. In another aspect the present invention relates to a pharmaceutical composition which does not contain any pharmaceutically acceptable excipient in addition to therapeutically effective quantities of **1** and **2.**

The present invention also relates to the use of **1** and **2** for preparing a pharmaceutical composition containing therapeutically effective quantities of **1** and **2** for treating inflammatory and/or obstructive diseases of the respiratory tract, particularly asthma or chronic obstructive pulmonary disease (COPD), and complications thereof such as pulmonary hypertension, as well as allergic and non-allergic rhinitis.

The present invention also relates to the use of a pharmaceutical formulation containing a compound of formula **1** for preparing a pharmaceutical composition for treating inflammatory and/or obstructive diseases of the respiratory tract, particularly asthma or chronic obstructive pulmonary disease (COPD), as well as complications thereof such as pulmonary hypertension, as well as allergic and non-allergic rhinitis, characterised in that the pharmaceutical formulation contains a compound of formula **2.**

This present invention also relates to the prophylaxis of, treating of, or reducing the exacerbations associated with pulmonary diseases, in particular inflammatory or obstructive diseases of the respiratory tract, by administering to a patient in need thereof an effective amount of a pharmaceutical composition according to this invention either in a single combined form, separately, or separately and sequentially where the sequential administration is close in time, or remote in time.

In addition, the present invention also relates to the use of a compound of the formula **1** in the prophylaxis of, treating of, or reducing the exacerbations associated with pulmonary diseases, in particular inflammatory or obstructive diseases of the respiratory tract, by administering to a patient in need thereof an effective amount of the compound of the formula 1 in combination with a compound of the formula **2** either in a single combined form, separately, or separately and sequentially where the sequential administration is close in time, or remote in time.

Furthermore, the present invention also relates to the use of a compound of the formula **2** in the prophylaxis of, treating of, or reducing the exacerbations associated with pulmonary diseases, in particular inflammatory or obstructive diseases of the respiratory tract, by administering to a patient in need thereof an effective amount of a compound of the formula **1** in combination with the compound of the formula **2** either in a single combined form, separately, or separately and sequentially where the sequential administration is close in time, or remote in time.

Consequently, the present invention also relates to the use of a compound of the formula **1** in the manufacture of a medicament for the prophylaxis of, treating of, or reducing the exacerbations associated with pulmonary diseases, in particular inflammatory or obstructive diseases of the respiratory tract, by administering to a patient in need thereof an effective amount of the compound of the formula **1** in combination with a compound of the formula **2** either in a single combined form, separately, or separately and sequentially where the sequential administration is close in time, or remote in time.

Furthermore consequently, the present invention also relates to the use of a compound of the formula **2** in the manufacture of a medicament for the prophylaxis of, treating of, or reducing the exacerbations associated with pulmonary diseases, in particular inflammatory or obstructive diseases of the respiratory tract, by administering to a patient in need thereof an effective amount of a compound of the formula **1** in combination with the compound of the formula **2** either in a single combined form, separately, or separately and sequentially where the sequential administration is close in time, or remote in time.

The present invention also relates to the simultaneous or successive use of therapeutically effective doses of the active compounds **1** and **2** for treating inflammatory and/or obstructive diseases of the respiratory tract, particularly asthma or chronic obstructive pulmonary disease (COPD), and complications thereof such as pulmonary hypertension, as well as allergic and non-allergic rhinitis, by simultaneous or successive administration.

The therapeutic utility of the combinations of compounds of the present invention is applicable to a patient or subject afflicted with a disease or condition as herein set forth and therefore in need of such treatment. The beneficial results are therapeutic whether administered to animals or humans. As used herein the terms "animal" and "animals" is used merely for the purpose of pointing out human beings as opposed to other members of the animal kingdom. The combinations of compounds of the present invention have therapeutic applicability in the treatment of mammals, and in particular of humans. All of the major subdivisions of the class of mammals *(Mammalia)* are included within the scope of the present invention with regard to being recipients of therapeutic treatment as described herein. Mammals have value as pets to humans and are therefore likely to be subjects of treatment. This applies especially to the canine and feline groups of mammals. Other mammals are valued as domesticated animals and their treatment in accordance with the present invention is likely in view of the adverse economic impact of not treating the diseases and conditions described herein. This applies especially to the equine, bovine, porcine, and ovine groups of mammals.

The pharmaceutical composition of the present invention is useful in the treatment and/or prophylaxis of obstructive airways diseases, especially of asthma, chronic obstructive pulmonary disease (COPD) and other obstructive airways diseases exacerbated by heightened bronchial reflexes, inflammation, bronchial hyper-reactivity and bronchospasm, in particular COPD.

In particular, the combination of a compound of the formula **1** and a compound of the formula **2** are useful in the treatment of respiratory diseases and conditions comprising: asthma, acute respiratory distress syndrome, chronic pulmonary inflammatory disease, bronchitis, chronic bronchitis, chronic obstructive pulmonary (airway) disease, including the pulmonary symptoms associated with cystic fibrosis and mucoviscidosis; and silicosis; or immune diseases and conditions comprising: allergic rhinitis and chronic sinusitis.

The types of diseases that may be treated using the combination of an anticholinergic of the formula **1** and a compound of the formula **2** include but are not limited to asthma; chronic or acute bronchoconstriction; chronic bronchitis; small airways obstruction; emphysema; chronic obstructive pulmonary disease (COPD); COPD that has chronic bronchitis, pulmonary emphysema or dyspnea associated therewith; COPD that is characterized by irreversible, progressive airways obstruction; adult respiratory distress syndrome (ARDS); exacerbation of airways hyper-reactivity consequent to drug therapy; pneumoconiosis; acute bronchitis; acute laryngotracheal bronchitis; arachidic bronchitis; catarrhal bronchitis; croupus bronchitis; dry bronchitis; infectious asthmatic bronchitis; productive bronchitis; staphylococcus or streptococcal bronchitis; vesicular bronchitis; cylindric bronchiectasis; sacculated bronchiectasis; fusiform bronchiectasis; capillary bronchiectasis; cystic bronchiectasis; dry bronchiectasis; follicular bronchiectasis; seasonal allergic rhinitis; perennial allergic rhinitis; purulent or nonpurulent sinusitis; acute or chronic sinusitis; ethmoid, frontal, maxillary, or sphenoid sinusitis; eosinophilia; pulmonary infiltration eosinophilia; Loffler's syndrome; chronic eosinophilic pneumonia; tropical pulmonary eosinophilia; bronchopneumonic aspergillosis; aspergilloma; granulomas containing eosinophils; allergic granulomatous angiitis or Churg-Strauss syndrome; sarcoidosis; alveolitis; chronic hypersensitivity pneumonitis; diffuse interstitial pulmonary fibrosis or interstitial lung fibrosis; and idiopathic pulmonary fibrosis.

One of the important respiratory diseases treatable with the combinations of therapeutic agents of the present invention is asthma, a chronic, increasingly common disorder encountered worldwide and characterized by intermittent reversible airway obstruction, airway hyper-responsiveness and inflammation. The cause of asthma has yet to be determined, but the most common pathological expression of asthma is inflammation of the airways, which may be significant even in the airways of patients with mild asthma. Based on bronchial biopsy and lavage studies it has been clearly shown that asthma involves infiltration by mast cells, eosinophils, and T-lymphocytes into a patient's airways. Bronchoalveolar lavage (BAL) in atopic asthmatics shows activation of interleukin (IL)-3, IL-4, IL-5 and granulocyte/macrophage-colony stimulating factor (GM-CSF) that suggests the presence of a T-helper 2 (Th-2)-like T-cell population.

The combinations of therapeutic agents of the present invention are useful in the treatment of atopic and non-atopic asthma. The term "atopy" refers to a genetic predisposition toward the development of type I (immediate) hypersensitivity reactions against common environmental antigens. The most common clinical manifestation is allergic rhinitis, while bronchial asthma, atopic dermatitis, and food allergy occur less frequently. Accordingly, the expression "atopic asthma" as used herein is intended to be synonymous with "allergic asthma", *i.e.,* bronchial asthma which is an allergic manifestation in a sensitized person. The term "non-atopic asthma" as used herein is intended to refer to all other asthmas, especially essential or "true" asthma, which is provoked by a variety of factors, including vigorous exercise, irritant particles, psychologic stresses, *etc*.

A single dose of the combination of the active substances **1** and **2** according to this invention is administered to the patient, preferably a human, in need of such treatment and/or prophylaxis once, twice, three times or more per day, preferably one or twice, most preferably once per day. Preferably the two active substances **1** and **2** are co-administered, more preferably co-administered as a combined fixed dose, but these active substances may also be administered sequentially, whereby sequentially is preferably close in time, preferably the time gap between an administration of a compound **1** and a compound **2** or vice versa is 3 hours or less, more preferably 1 hour or less.

In the active substance combinations of **1** and **2** according to the invention, ingredients **1** and **2** may be present in the form of their enantiomers, mixtures of enantiomers or in the form of racemates.

The proportions in which the two active substances **1** and **2** may be used in the active substance combinations according to the invention are variable. Active substances **1** and **2** may possibly be present in the form of their solvates or hydrates. Depending on the choice of the compounds **1** and **2**, the weight ratios which may be used within the scope of the present invention vary on the basis of the different molecular weights of the various compounds and their different potencies.

As a rule, the pharmaceutical combinations according to the invention may contain compounds **1** and **2** in ratios by weight ranging from 1:4000 to 8:1, preferably from 1:1000 to 1:1.2, more preferably from 1:1000 to 1:2,5, wherein the ratio is based on the weight of the corresponding cation **1'** of **1.**

In the preferred combination of a compound **1a** and the compound **2** according to this invention, the lower limit of the weight ratio of **1a** to **2**, calculated as the amount of the tiotropiumcation **1**' to **2**, is preferably 1:4000, more preferably 1:1000, most preferably 1:361; whereas the upper limit of the weight ratio of **1a** to **2**, calculated as the amount of the corresponding cation **1'** of **1a** to **2**, is preferably 1:2.5, more preferably 1:12.5, most preferably 1:24. The corresponding lower and upper limits with regard to tiotropium salts, especially tiotropium bromide, and/or hydrates, in particular tiotropium bromide monohydrate, can be calculated accordingly based on the corresponding molecular weights.

In the preferred combination of a compound **1b** and the compound **2** according to this invention, the lower limit of the weight ratio of **1b** to **2**, calculated as the amount of the corresponding cation **1'** of **1b** to **2**, is preferably 1:4000, more preferably 1:1000, most preferably 1:361; whereas the upper limit of the weight ratio of **1b** to **2**, calculated as the amount of the corresponding cation **1'** of **1b** to **2**, is preferably 8:1, more preferably 1:1.2. The corresponding lower and upper limits with regard to salts of **1b**, especially the bromide, and/or hydrates can be calculated accordingly based on the corresponding molecular weights.

For example, without restricting the scope of the invention thereto, preferred combinations of **1a** and **2** according to the invention may contain tiotropium **1'** and the compound of formula **2** in the following weight ratios: 1:361, 1:350, 1:340, 1:330, 1:320, 1:310, 1:290, 1:280, 1:270, 1:260, 1:250, 1:240, 1:230, 1:220, 1:210, 1:200, 1:190, 1:180, 1:170, 1:160, 1:150, 1:140, 1:130, 1:120, 1: 110, 1:100, 1:90, 1:80, 1:70, 1:60, 1:50, 1:40, 1:30, 1:24.

For example, without restricting the scope of the invention thereto, preferred combinations of **1b** and **2** according to the invention may contain the cation **1'** of **1b** and the compound of formula **2** in the following weight ratios: 1:361, 1:350, 1:340, 1:330, 1:320, 1:310, 1:290, 1:280, 1:270, 1:260, 1:250, 1:240, 1:230, 1:220, 1:210, 1:200, 1:190, 1:180, 1:170, 1:160, 1:150, 1:140, 1:130, 1:120, 1:110, 1:100, 1:90, 1:80, 1:70, 1:60, 1:50, 1:40, 1:30, 1:20, 1:10, 1:5, 1:3, 1:1.2.

The pharmaceutical compositions according to the invention containing the combinations of **1** and **2** are normally administered so that **1** and **2** are present together in doses of 25 to 10000 µg, preferably from 100 to 5800 µg, more preferably from 500 to 3400 µg per single dose.

Combinations of **1a** and **2** according to the invention contain a quantity of tiotropium **1'** and compound of formula **2** such that the lower limit of the total dosage per single dose is preferably 27.5 µg, more preferably 105 µg, most preferably 508 µg; whereas the upper limit of the total dosage per single dose is preferably 10000µg, more preferably 5040µg, most preferably 3021 µg.

For example, combinations of **1a** and **2** according to the invention contain a quantity of tiotropium **1'** and compound of formula **2** such that the total dosage per single dose is about 508µg, 510µg, 515µg, 520µg, 525µg, 530µg, 535µg, 540µg, 545µg, 550µg, 555µg, 560µg, 565µg, 570µg, 575µg, 580µg, 585µg, 590µg, 595µg, 600µg, 605µg, 610µg, 615µg, 620µg, 625µg, 630µg, 635µg, 640µg, 645µg, 650µg, 655µg, 660µg, 665µg, 670µg, 675µg, 680µg, 685µg, 690µg, 695µg, 700µg, 705µg, 710µg, 715µg, 720µg, 725µg, 730µg, 735µg, 740µg, 745µg, 750µg, 755µg, 760µg, 765µg, 770µg, 775µg, 780µg, 785µg, 790µg, 795µg, 800µg, 805µg, 810µg, 815µg, 820µg, 825µg, 830µg, 835µg, 840µg, 845µg, 850µg, 855µg, 860µg, 865µg, 870µg, 875µg, 880µg, 885µg, 890µg, 895µg, 900µg, 905µg, 910µg, 915µg, 920µg, 925µg, 930µg, 935µg, 940µg, 945µg, 950µg, 955µg, 960µg, 965µg, 970µg, 975µg, 980µg, 985µg, 990µg, 995µg, 1000µg, 1050µg, 1150µg, 1100µg, 1200µg, 1250µg, 1300µg, 1350µg, 1400µg, 1450µg, 1500µg, 1550µg, 1600µg, 1650µg, 1700µg, 1750µg, 1800µg, 1850µg, 1900µg, 1950µg, 2000µg, 2050µg, 2100µg, 2150µg, 2200µg, 2250µg, 2300µg, 2350µg, 2400µg, 2450µg, 2500µg, 2550µg, 2600µg, 2650µg, 2700µg, 2750µg, 2800µg, 2850µg, 2900µg, 2950µg, 3000µg, 3050µg, 3100µg or similar. The suggested dosages per single dose specified above are not to be regarded as being limited to the numerical values actually stated, but are intended as dosages which are disclosed by way of example. Of course, dosages which fluctuate about the abovementioned numerical values within a range of about +/- 25 µg are also included in the values given above by way of example. In these dosage ranges, the active substances **1'** and **2** may be present in the weight ratios given above.

Combinations of **1b** and **2** according to the invention contain a quantity of the cation **1'** of **1b** and compound of formula **2** such that the lower limit of the total dosage per single dose is preferably 27.5 µg, more preferably 105 µg, most preferably 508 µg; whereas the upper limit of the total dosage per single dose is preferably 10800 µg, more preferably 5412 µg, most preferably 3412 µg.

For example, combinations of **1b** and **2** according to the invention contain a quantity of the cation **1'** of **1b** and compound of formula **2** such that the total dosage per single dose is about 508µg, 510µg, 515µg, 520µg, 525µg, 530µg, 535µg, 540µg, 545µg, 550µg, 555µg, 560µg, 565µg, 570µg, 575µg, 580µg, 585µg, 590µg, 595µg, 600µg, 605µg, 610µg, 615µg, 620µg, 625µg, 630µg, 635µg, 640µg, 645µg, 650µg, 655µg, 660µg, 665µg, 670µg, 675µg, 680µg, 685µg, 690µg, 695µg, 700µg, 705µg, 710µg, 715µg, 720µg, 725µg, 730µg, 735µg, 740µg, 745µg, 750µg, 755µg, 760µg, 765µg, 770µg, 775µg, 780µg, 785µg, 790µg, 795µg, 800µg, 805µg, 810µg, 815µg, 820µg, 825µg, 830µg, 835µg, 840µg, 845µg, 850µg, 855µg, 860µg, 865µg, 870µg, 875µg, 880µg, 885µg, 890µg, 895µg, 900µg, 905µg, 910µg, 915µg, 920µg, 925µg, 930µg, 935µg, 940µg, 945µg, 950µg, 955µg, 960µg, 965µg, 970µg, 975µg, 980µg, 985µg, 990µg, 995µg, 1000µg, 1050µg, 1150µg, 1100µg, 1200µg, 1250µg, 1300µg, 1350µg, 1400µg, 1450µg, 1500µg, 1550µg, 1600µg, 1650µg, 1700µg, 1750µg, 1800µg, 1850µg, 1900µg, 1950µg, 2000µg, 2050µg, 2100µg, 2150µg, 2200µg, 2250µg, 2300µg, 2350µg, 2400µg, 2450µg, 2500µg, 2550µg, 2600µg, 2650µg, 2700µg, 2750µg, 2800µg, 2850µg, 2900µg, 2950µg, 3000µg, 3050µg, 3100µg, 3150µg, 3200µg, 3250µg, 3300µg, 3350µg, 3400µg, 3410µg or similar. The suggested dosages per single dose specified above are not to be regarded as being limited to the numerical values actually stated, but are intended as dosages which are disclosed by way of example. Of course, dosages which fluctuate about the abovementioned numerical values within a range of about +/- 25 µg are also included in the values given above by way of example. In these dosage ranges, the active substances **1'** and **2** may be present in the weight ratios given above.

In a combination of the active substances **1a** and **2** according to this invention, the lower limit.of the quantity of active substance **1a** calculated as the amount of tiotropium **1'** administered per single dose is preferably 2.5 µg, more preferably 5 *µ*g, most preferably 8.3 µg; whereas the upper limit of the quantity of active substance **1a** calculated as the amount of tiotropium **1'** administered per single dose is preferably 40 µg, more preferably 20.8 µg. With respect to certain diseases, especially such diseases where a thick mucus layer will impair penetration of the active substances to the target receptor, like e.g. cystic fibrosis, higher quantities of tiotropium, in particular up to about 80 µg may be beneficial. The corresponding lower and upper limits with regard to other tiotropium salts, especially tiotropium bromide, and/or hydrates, in particular tiotropium bromide hydrate, can be calculated accordingly based on the corresponding molecular weights.

In a combination of the active substances **1a** and **2** according to this invention, the lower limit of the quantity of active substance **2** administered per single dose is preferably 25 µg, more preferably 100 µg, most preferably 500 µg; whereas the upper limit of the quantity of active substance 2 administered per single dose is preferably 10000µg, more preferably 5000µg, most preferably 3000 µg. The corresponding lower and upper limits with regard to other salts and/or hydrates of **2** can be calculated accordingly based on the corresponding molecular weights.

For example, without restricting the scope of the invention thereto, according to the first embodiment of this invention the combinations of **1a** and **2** may contain a quantity of tiotropium **1'** and the compound of formula **2** such that, for each single dose, 8.3 µg of **1'** and 500 µg of **2**, 10 µg of **1'** and 500 µg of **2**, 12 µg of **1'** and 500 µg of **2,** 14µg of **1'** and 500 µg of **2,** 16µg of **1'** and 500 µg of **2,** 18 µg of **1'** and 500 µg of **2**, 20.8 µg of **1'** and 500µg of **2**,
8.3 µg of **1**' and 750 µg of **2**, 10 µg of **1'** and 750 µg of **2**, 12 µg of **1'** and 750 µg of **2**, 14 µg of **1'** and 750 µg of **2**, 16 µg of **1'** and 750 µg of **2**, 18 µg of **1'** and 750 µg of **2**, 20.8 µg of **1'** and 750 µg of **2**,
8.3 µg of **1'** and 1000 µg of **2**, 10 µg of **1'** and 1000 µg of **2**, 12 µg of **1'** and 1000µg of **2**, 14 µg of **1'** and 1000 µg of **2**, 16 µg of **1'** and 1000 µg of **2**, 18 µg of **1'** and 1000 µg of **2**, 20.8 µg of **1'** and 1000 µg of **2**,
8.3µg of **1'** and 1250µg of **2**, 10 µg of **1'** and 1250 µg of **2**, 12 µg of **1'** and 1250 µg of **2**, 14 µg of **1'** and 1250 µg of **2**, 16 µg of **1'** and 1250 µg of **2**, 18 µg of **1'** and 1250 µg of **2**, 20.8 µg of **1'** and 1250 µg of **2**,
8.3 µg of **1'** and 1500 µg of **2**, 10 µg of **1'** and 1500 µg of **2**, 12 µg of **1'** and 1500µg of **2**, 14µg of **1'** and 1500µg of **2**, 16 µg of **1'** and 1500 µg of **2**, 18 µg of **1'** and 1500 µg of **2**, 20.8 µg of **1'** and 1500 µg of **2**,
8.3 µg of **1'** and 1750µg of **2**, 10µg of **1'** and 1750 µg of **2**, 12µg of **1'** and 1750 µg of **2** , 14 µg of **1'** and 1750 µg of **2**, 16 µg of **1'** and 1750 µg of **2**, 18 µg of **1'** and 1750 µg of **2**, 20.8 µg of **1'** and 1750µg of **2**,
8.3 µg of **1'** and 2000 µg of **2**, 10 µg of **1'** and 2000 µg of **2**, 12 µg of **1'** and 2000 µg of **2**, 14 µg of **1'** and 2000 µg of **2**, 16 µg of **1'** and 2000 µg of **2**, 18 µg of **1'** and 2000 µg of **2**, 20.8 µg of **1'** and 2000 µg of **2**,
8.3 µg of **1'** and 2250 µg of **2**, 10 µg of **1'** and 2250 µg of **2**, 12 µg of **1'** and 2250 µg of **2**, 14 µg of **1'** and 2250 µg of **2**, 16 µg of **1'** and 2250 µg of **2**, 18 µg of **1'** and 2250 µg of **2**, 20.8 µg of **1'** and 2250 µg of **2**,
8.3 µg of **1'** and 2500 µg of **2**, 10 µg of **1'** and 2500 µg of **2**, 12 µg of **1'** and 2500 µg of **2**, 14 µg of **1'** and 2500 µg of **2**, 16 µg of **1'** and 2500 µg of **2**, 18 µg of **1'** and 2500 µg of **2**, 20.8 µg of **1'** and 2500 µg of **2**,
8.3 µg of **1'** and 2750 µg of **2**, 10 µg of **1'** and 2750 µg of **2**, 12 µg of **1'** and 2750 µg of **2**, 14 µg of **1'** and 2750 µg of **2**, 16 µg of **1'** and 2750 µg of **2**, 18 µg of **1'** and 2750 µg of **2**, 20.8 µg of **1'** and 2750 µg of **2**,
8.3 µg of **1'** and 3000 µg of **2**, 10 µg of **1'** and 3000 µg of **2**, 12 µg of **1'** and 3000 µg of **2**, 14 µg of **1'** and 3000 µg of **2**, 16 *µ*g of **1'** and 3000 µg of **2**, 18 µg of **1'** and 3000 µg of **2**, 20.8 µg of **1'** and 3000 µg of **2** are administered. The corresponding quantities with regard to tiotropium salts **1a**, especially tiotropium bromide, and/or hydrates, in particular tiotropium bromide monohydrate, can be calculated accordingly based on the corresponding molecular weights.

In a combination of the active substances **1b** and **2** according to this invention, the lower limit of the quantity of active substance **1b** calculated as the amount of the corresponding cation **1'** administered per single dose is preferably 2.5 µg, more preferably 5 µg, most preferably 8.3 µg; whereas the upper limit of the quantity of active substance **1b** calculated as the amount of the corresponding cation **1'** of **1** administered per single dose is preferably 800 µg, more preferably 412.8 µg. The corresponding lower and upper limits with regard to salts of **1b,** especially the bromide, and/or hydrates can be calculated accordingly based on the corresponding molecular weights.

In a combination of the active substances **1b** and **2** according to this invention, the lower limit of the quantity of active substance **2** administered per single dose is preferably 25 µg, more preferably 100 µg, most preferably 500 µg; whereas the upper limit of the quantity of active substance **2** administered per single dose is preferably 10000µg, more preferably 5000µg, most preferably 3000µg. The corresponding lower and upper limits with regard to other salts and/or hydrates of **2** can be calculated accordingly based on the corresponding molecular weights.

For example and without restricting the scope of the invention thereto, according to the second embodiment of this invention the combinations of **1b** and **2** may contain an amount of the corresponding cation **1'** of **1b** and the compound of formula **2** such that 8.3µg of **1'** and 25µg of **2**, 8.3µg of **1'** and 50µg of **2**, 8.3µg of **1'** and 100µg of **2**, 8.3µg of **1'** and 200µg of **2**, 8.3µg of **1'** and 300µg of **2**, 8.3µg of **1'** and 400µg of **2**, 8.3µg of **1'** and 500µg of **2**, 8.3µg of **1'** and 600µg of **2**, 8.3µg of **1'** and 700µg of **2**, 8.3µg of **1'** and 800µg of **2**, 8.3µg of **1'** and 900µg of **2**, 8.3µg of **1'** and 1000µg of **2**, 8.3µg of **1'** and 11 00µg of **2**, 8.3µg of **1'** and 1200µg of **2**, 8.3,µg of **1'** and 1300µg of **2**, 8.3µg of **1'** and 1400µg of **2**, 8.3µg of **1'** and 1500µg of **2**, 8.3µg of **1'** and 1600µg of **2**, 8.3µg of **1'** and 1700µg of **2**, 8.3µg of **1'** and 1800µg of **2**, 8.3µg of **1'** and 1900µg of **2**, 8.3µg of **1'** and 2000µg of **2**, 8.3µg of **1'** and 2100µg of **2**, 8.3µg of **1'** and 2200µg of **2**, 8.3µg of **1'** and 2300µg of **2**, 8.3µg of **1'** and 2400µg of **2**, 8.3µg of **1'** and 2500µg of **2**, 8.3µg of **1'** and 2600µg of **2**, 8.3µg of **1'** and 2700µg of **2**, 8.3µg of **1'** and 2800µg of **2**, 8.3µg of **1'** and 2900µg of **2**, 8.3µg of **1'** and 3000µg of **2**,
16.5µg of **1'** and 25µg of **2**, 16.5µg of **1'** and 50µg of **2**, 16.5µg of **1'** and 100µg of **2**, 1 6.5µg of **1'** and 200µg of **2**, 1 6.5µg of **1'** and 300µg of **2**, 1 6.5µg of **1'** and 400µg of **2**, 16.5µg of **1'** and 500µg of **2**, 16.5µg of **1'** and 600µg of **2**, 16.5µg of **1'** and 700µg of **2**, 16.5µg of **1'** and 800µg of **2**,16.5µg of **1'** and 900µg of **2**, 16.5µg of **1'** and 1000µg of **2**, 16.5µg of **1'** and 1100µg of **2**, 16.5µg of **1'** and 1200µg of **2**, 16.5µg of **1'** and 1300µg of **2**, 16.5µg of **1'** and 1400*µ*g of **2**, 16.5µg of **1'** and 1500µg of **2**, 16.5µg of **1'** and 1600µg of **2**, 16.5µg of **1'** and 1700µg of **2**, 16.5µg of **1'** and 1800µg of **2**, 16.5µg of **1'** and 1900µg of **2**, 16.5µg of **1'** and 2000µg of **2**, 16.5µg of **1'** and 2100µg of **2**, 16.5µg of **1'** and 2200µg of **2**, 16.5µg of **1'** and 2300µg of **2**, 16.5µg of **1'** and 2400µg of **2,** 16.5µg of **1'** and 2500µg of **2**, 16.5µg of **1'** and 2600µg of **2**, 16.5µg of **1'** and 2700µg of **2**, 16.5µg of **1'** and 2800µg of **2**, 16.5µg of **1'** and 2900µg of **2**, 16.5µg of **1'** and 3000µg of **2,**
33µg of **1'** and 25µg of **2**, 33µg of **1'** and 50µg of **2**, 33µg of **1'** and 100µg of **2**, 33µg of **1'** and 200µg of **2**, 33µg of **1'** and 300µg of **2**, 33µg of **1'** and 400µg of **2**, 33µg of **1'** and 500µg of **2**, 33µg of **1'** and 600µg of **2**, 33µg of **1'** and 700µg of **2**, 33µg of **1'** and 800µg of **2**, 33µg of **1'** and 900µg of **2**, 33µg of **1'** and 1000µg of **2**, 33µg of **1'** and 1100µg of **2**, 33µg of **1'** and 1200µg of **2**, 33µg of **1'** and 1300µg of **2**, 33µg of **1'** and 1400µg of **2**, 33µg of **1'** and 1500µg of **2**, 33µg of **1'** and 1600µg of **2**, 33µg of **1'** and 1700µg of **2**, 33µg of **1'** and 1800µg of **2**, 33µg of **1'** and 1900µg of **2**, 33µg of **1'** and 2000µg of **2**, 33µg of **1'** and 2100µg of **2**, 33µg of **1'** and 2200µg of **2**, 33µg of **1'** and 2300µg of **2**, 33µg of **1'** and 2400µg of **2**, 33µg of **1'** and 2500µg of **2**, 33µg of **1'** and 2600µg of **2**, 33µg of **1'** and 2700µg of **2**, 33µg of **1'** and 2800µg of **2**, 33µg of **1'** and 2900µg of **2**, 33µg of **1'** and 3000µg of **2**,
49.5µg of **1'** and 25µg of **2**, 49.5µg of **1'** and 50µg of **2**, 49.5µg of **1'** and 100µg of **2**, 49.5µg of **1'** and 200µg of **2**, 49.5µg of **1'** and 300µg of **2**, 49.5µg of **1'** and 400µg of **2**, 49.5µg of **1'** and 500µg of **2**, 49.5µg of **1'** and 600µg of **2**, 49.5µg of **1'** and 700µg of **2**, 49.5µg of **1'** and 800µg of **2**, 49.5µg of **1'** and 900µg of **2**, 49.5µg of **1'** and 1000µg of **2**, 49.5µg of **1'** and 1100µg of **2**, 49.5µg of **1'** and 1200µg of **2**, 49.5µg of **1'** and 1300µg of **2**, 49.5µg of **1'** and 1400*µ*g of **2**, 49.5µg of **1'** and 1500µg of **2**, 49.5µg of **1'** and 1600µg of **2**, 49.5µg of **1'** and 1700µg of **2**, 49.5µg of **1'** and 1800µg of **2**, 49.5µg of **1'** and 1900µg of **2**, 49.5µg of **1'** and 2000µg of **2**, 49.5µg of **1'** and 2100µg of **2**, 49.5µg of **1'** and 2200µg of **2**, 49.5µg of **1'** and 2300µg of **2**, 49.5µg of **1'** and 2400µg of **2**, 49.5µg of **1'** and 2500µg of **2**, 49.5µg of **1'** and 2600µg of **2**, 49.5µg of **1'** and 2700µg of **2**, 49.5µg of **1'** and 2800µg of **2**, 49.5µg of **1'** and 2900µg of **2**, 49.5µg of **1'** and 3000µg of **2**,
82.6µg of **1'** and 25µg of **2**, 82.6µg of **1'** and 50µg of **2**, 82.6µg of **1'** and 100µg of **2**, 82.6µg of **1'** and 200µg of **2**, 82.6µg of **1'** and 300µg of **2**, 82.6µg of **1'** and 400µg of **2**, 82.6µg of **1'** and 500µg of **2**, 82.6µg of **1'** and 600µg of **2,** 82.6µg of **1'** and 700µg of **2**, 82.6µg of **1'** and 800µg of **2**, 82.6µg of **1'** and 900µg of **2**, 82.6µg of **1'** and 1000µg of **2**, 82.6µg of **1'** and 1100µg of **2**, 82.6µg of **1'** and 1200µg of **2**, 82.6µg of **1'** and 1300µg of **2**, 82.6µg of **1'** and 1400µg of **2,** 82.6µg of **1'** and 1500µg of **2**, 82.6µg of **1'** and 1600µg of **2**, 82.6µg of **1'** and 1700µg of **2**, 82.6µg of **1'** and 1800µg of **2**, 82.6µg of **1'** and 1900µg of **2**, 82.6µg of **1'** and 2000µg of **2**, 82.6µg of **1'** and 2100µg of **2,** 82.6µg of **1'** and 2200µg of **2**, 82.6µg of **1'** and 2300µg of **2**, 82.6µg of **1'** and 2400µg of **2**, 82.6µg of **1'** and 2500µg of **2**, 82.6µg of **1'** and 2600µg of **2**, 82.6µg of **1'** and 2700µg of **2**, 82.6µg of **1'** and 2800µg of **2**, 82.6µg of **1'** and 2900µg of **2**, 82.6µg of **1'** and 3000µg of **2**,
165.1µg of **1'** and 25µg of **2**, 165.1,ug of **1'** and 50µg of **2**, 165.1µg of **1'** and 100µg of **2**, 165.1µg of **1'** and 200µg of **2**, 165.1µg of **1'** and 300µg of **2**, 165.1µg of **1'** and 400µg of **2**, 165.1µg of **1'** and 500µg of **2**, 165.1µg of **1'** and 600µg of **2**, 165.1µg of **1'** and 700µg of **2**, 165.1µg of **1'** and 800µg of **2**, 165.1µg of **1'** and 900µg of **2**, 165.1µg of **1'** and 1000µg of **2**, 165.1µg of **1'** and 1100µg of **2**, 165.1µg of **1'** and 1200µg of **2,** 165.1µg of **1'** and 1300µg of **2**, 165.1µg of **1'** and 1400µg of **2,** 165.1µg of **1'** and 1500µg of **2**, 165.1µg of **1'** and 1600µg of **2,**, 165.1µg of **1'** and 1700µg of **2**, 165.1µg of **1** and 1800µg of **2**, 165.1µg of **1'** and 1900µg of **2**, 165.1µg of **1'** and 2000µg of **2**, 165.1µg of **1'** and 2100µg of **2**, 165.1µg of **1'** and 2200µg of **2**,165.1µg of **1'** and 2300µg of **2**, 165.1µg of **1'** and 2400µg of **2,** 165.1µg of **1'** and 2500µg of **2**, 165.1µg of **1'** and 2600µg of **2**, 165.1µg of **1'** and 2700µg of **2**, 165.1µg of **1'** and 2800µg of **2**, 165.1µg of **1'** and 2900µg of **2**, 165.1µg of **1'** and 3000µg of **2**,
206,4,µg of **1'** and 25µg of **2**, 206.4µg of **1'** and 50µg of **2**, 206.4µg of **1'** and 100µg of **2**, 206.4µg of **1'** and 200µg of **2**, 206.4µg of **1'** and 300µg of **2**, 206.4µg of **1'** and 400µg of **2**, 206.4µg of **1'** and 500µg of **2**, 206.4µg of **1'** and 600µg of **2**, 206.4µg of **1'** and 700µg of **2**, 206.4µg of **1'** and 800µg of **2**, 206.4µg of **1'** and 900µg of **2**, 206.4µg of **1'** and 1 000µg of **2**, 206.4µg of **1'** and 1100µg of **2**, 206.4,ug of **1'** and 1200µg of **2**, 206.4µg of **1'** and 1300µg of **2**, 206.4µg of **1'** and 1400µg of **2**, 206.4µg of **1'** and 1500µg of **2**, 206.4µg of **1'** and 1600µg of **2**, 206.4µg of **1'** and 1700µg of **2**, 206.4µg of **1'** and 1800µg of **2**, 206.4µg of **1'** and 1900µg of **2**, 206.4µg of **1'** and 2000µg of **2**, 206.4µg of **1'** and 2100µg of **2**, 206.4µg of **1'** and 2200µg of **2**, 206.4µg of **1'** and 2300µg of **2**, 206.4µg of **1'** and 2400µg of **2**, 206.4µ/g of **1'** and 2500µg of **2**, 206.4µg of **1'** and 2600µg of **2**, 206.4µg of **1'** and 2700µg of **2**, 206.4µg of **1'** and 2800µg of **2**, 206.4µg of **1'** and 2900µg of **2**, 206.4µg of **1'** and 3000µg of **2**,
412.8µg of **1'** and 25µg of **2**, 412.8µg of **1'** and 50µg of **2**, 412.8µg of **1'** and 100µg of **2**, 412.8µg of **1'** and 200µg of **2**, 412.8µg of **1'** and 300µg of **2**, 412.8µg of **1'** and 400µg of **2**, 412.8µg of **1'** and 500µg of **2** or 412.8µg of **1'** and 600µg of **2**, 412.8µg of **1'** and 700µg of **2**, 412.8µg of **1'** and 800µg of **2**, 412.8µg of **1'** and 900µg of **2**, 412.8µg of **1'** and 1000µg of **2,** 412.8µg of **1'** and 1100µg of **2**, 412.8µg of **1'** and 1200µg, of **2**, 412.8µg of **1'** and 1300µg of **2**, 412.8µg of **1'** and 1400µg of **2**, 412.8µg of **1'** and 1500µg of **2**, 412.8µg of **1'** and 1600µg of **2**, 412.8µg of **1'** and 1700µg of **2**, 412.8µg of **1'** and 1800µg of **2**, 412.8µg of **1'** and 1900µg of **2**, 412.8µg of **1'** and 2000µg of **2**, 412.8µg of **1'** and 2100µg of **2**, 412.8µg of **1'** and 2200µg of **2**, 412.8µg of **1'** and 2300µg of **2**, 412.8µg of **1'** and 2400µg of **2**, 412.8µg of **1'** and 2500µg of **2**, 412.8µg of **1'** and 2600µg of **2**, 412.8µg of **1'** and 2700µg of **2**, 412.8µg of **1'** and 2800µg of **2**, 412.8µg of **1'** and 2900µg of **2**, 412.8µg of **1'** and 3000µg of **2**, are administered per single dose. The corresponding quantities with regard to salts of **1b**, especially the bromide, and/or hydrates can be calculated accordingly based on the corresponding molecular weights.

The active substance combinations of **1** and **2** according to the invention are preferably administered by inhalation. For this purpose, ingredients **1** and **2** have to be made available in forms suitable for inhalation. Inhalable preparations include inhalable powders, propellant-containing metering aerosols or propellant-free inhalable solutions. Inhalable powders according to the invention containing the combination of active substances **1** and **2** may consist of the active substances on their own or of a mixture of the active substances with physiologically acceptable excipients. Within the scope of the present invention, the term propellant-free inhalable solutions also includes concentrates or sterile inhalable solutions ready for use. The preparations according to the invention may contain the combination of active substances **1** and **2** either together in one formulation or in two separate formulations. These formulations which may be used within the scope of the present invention are described in more detail in the next part of the specification.

### A) Inhalable powder containing the combinations of active substances 1 and 2 according to the invention:

The inhalable powders according to the invention may contain **1** and **2** either on their own or in admixture with suitable physiologically acceptable excipients.

If the active substances **1** and **2** are present in admixture with physiologically acceptable excipients, the following physiologically acceptable excipients may be used to prepare these inhalable powders according to the invention: monosaccharides (e.g. glucose or arabinose), disaccharides (e.g. lactose, saccharose, maltose, trehalose), oligo- and polysaccharides (e.g. dextran), cyclodextrines (e.g. α-cyclodextrine, β-cyclodextrine, χ-cyclodextrine, methyl-β-cyclodextrine, hydroxypropyl-β-cyclodextrine), polyalcohols (e.g. sorbitol, mannitol, xylitol), salts (e.g. sodium chloride, calcium carbonate) or mixtures of these excipients with one another. Preferably, mono- or disaccharides are used, while the use of lactose or glucose is preferred, particularly, but not exclusively, in the form of their hydrates. For the purposes of the invention, lactose is the particularly preferred excipient, while lactose monohydrate is most particularly preferred.

Within the scope of the inhalable powders according to the invention the excipients have a maximum average particle size of up to 250µm, preferably between 10 and 150µm, most preferably between 15 and 80µm. It may sometimes seem appropriate to add finer excipient fractions with an average particle size of 1 to 9*µ*m to the excipient mentioned above. These finer excipients are also selected from the group of possible excipients listed hereinbefore. Finally, in order to prepare the inhalable powders according to the invention, micronised active substance **1** and **2**, preferably with an average particle size of 0.5 to 10µm, more preferably from 1 to 5*µ*m, is added to the excipient mixture. Processes for producing the inhalable powders according to the invention by grinding and micronising and finally mixing the ingredients together are known from the prior art. The inhalable powders according to the invention may be prepared and administered either in the form of a single powder mixture which contains both **1** and **2** or in the form of separate inhalable powders which contain only **1** or **2**.

The inhalable powders according to the invention may be administered using inhalers known from the prior art. Inhalable powders according to the invention which contain a physiologically acceptable excipient in addition to **1** and **2** may be administered, for example, by means of inhalers which deliver a single dose from a supply using a measuring chamber as described in US 4570630A, or by other means as described in DE 36 25 685 A. Preferably, the inhalable powders according to the invention which contain physiologically acceptable excipient in addition to **1** and **2** are packed into capsules (to produce so-called inhalettes) which are used in inhalers as described, for example, in WO 94/28958.

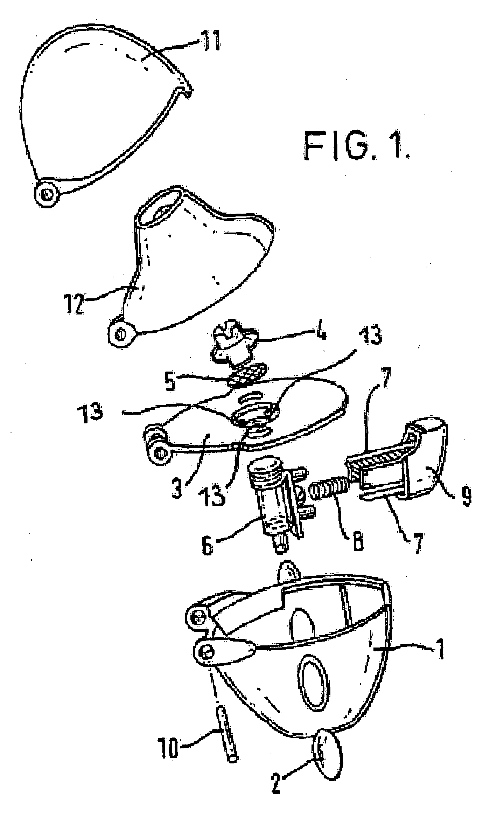

A particularly preferred inhaler for using the pharmaceutical combination according to the invention in inhalettes is shown in Figure 1.

This inhaler (Handyhaler) for inhaling powdered pharmaceutical compositions from capsules is characterised by a housing 1 containing two windows 2, a deck 3 in which there are air inlet ports and which is provided with a screen 5 secured via a screen housing 4, an inhalation chamber 6 connected to the deck 3 on which there is a push button 8 provided with two sharpened pins 7 and movable counter to a spring 8, and a mouthpiece 12 which is connected to the housing 1, the deck 3 and a cover 11 via a spindle 10 to enable it to be flipped open or shut, as well as air holes 13 for adjusting the flow resistance.

If the inhalable powders according to the invention are packed into capsules (inhalers) for the preferred use described above, the quantities packed into each capsule should be 1 to 30mg, preferably 3 to 20mg, more particularly 5 to 10mg of inhalable powder per capsule. These capsules contain, according to the invention, either together or separately, the doses of **1'** and **2** mentioned hereinbefore for each single dose.

### B) Propellant gas-driven inhalation aerosols containing the combinations of active substances 1 and 2:

Inhalation aerosols containing propellant gas according to the invention may contain substances **1** and **2** dissolved in the propellant gas or in dispersed form. **1** and **2** may be present in separate formulations or in a single preparation, in which **1** and **2** are either both dissolved, both dispersed or only one component is dissolved and the other is dispersed. The propellant gases which may be used to prepare the inhalation aerosols according to the invention are known from the prior art. Suitable propellant gases are selected from among hydrocarbons such as n-propane, n-butane or isobutane and halohydrocarbons such as preferably fluorinated derivatives of methane, ethane, propane, butane, cyclopropane or cyclobutane. The propellant gases mentioned above may be used on their own or in mixtures thereof. Particularly preferred propellant gases are halogenated alkane derivatives selected from TG 11, TG 12, TG 134a (1,1,1,2-tetrafluoroethane) and TG227 (1,1,1,2,3,3,3-heptafluoropropane) and mixtures thereof, of which the propellant gases TG134a, TG227 and mixtures thereof are preferred.

The propellant-driven inhalation aerosols according to the invention may also contain other ingredients such as co-solvents, stabilisers, surfactants, antioxidants, lubricants and pH adjusters. All these ingredients are known in the art.

The inhalation aerosols containing propellant gas according to the invention may contain up to 5 wt.-% of active substance **1** and/or **2**. Aerosols according to the invention contain, for example, 0.002 to 5 wt.-%, 0.01 to 3 wt.-%, 0.015 to 2 wt.-%, 0.1 to 2 wt.-%, 0.5 to 2 wt.-% or 0.5 to 1 wt.-% of active substance **1** and/or **2**.

If the active substances **1** and/or **2** are present in dispersed form, the particles of active substance preferably have an average particle size of up to 10µm, preferably from 0.1 to 5µm, more preferably from 1 to 5µm.

The propellant-driven inhalation aerosols according to the invention mentioned above may be administered using inhalers known in the art (MDIs = metered dose inhalers). Accordingly, in another aspect, the present invention relates to pharmaceutical compositions in the form of propellant-driven aerosols as hereinbefore described combined with one or more inhalers suitable for administering these aerosols. In addition, the present invention relates to inhalers which are characterised in that they contain the propellant gas-containing aerosols described above according to the invention.

The present invention also relates to cartridges which are fitted with a suitable valve and can be used in a suitable inhaler and which contain one of the above-mentioned propellant gas-containing inhalation aerosols according to the invention. Suitable cartridges and methods of filling these cartridges with the inhalable aerosols containing propellant gas according to the invention are known from the prior art.

### C) Propellant-free inhalable solutions or suspensions containing the combinations of active substances 1 and 2 according to the invention:

Propellant-free inhalable solutions and suspensions according to the invention contain, for example, aqueous or alcoholic, preferably ethanolic solvents, optionally ethanolic solvents mixed with aqueous solvents. If aqueous/ethanolic solvent mixtures are used the relative proportion of ethanol compared with water is not limited but the maximum is up to 70 percent by volume, more particularly up to 60 percent by volume and most preferably up to 30 percent by volume. The remainder of the volume is made up of water. The solutions or suspensions containing **1** and **2**, separately or together, are adjusted to a pH of 2 to 7, preferably 2 to 5, using suitable acids. The pH may be adjusted using acids selected from inorganic or organic acids. Examples of suitable inorganic acids include hydrochloric acid, hydrobromic acid, nitric acid, sulphuric acid and/or phosphoric acid. Examples of particularly suitable organic acids include ascorbic acid, citric acid, malic acid, tartaric acid, maleic acid, succinic acid, fumaric acid, acetic acid, formic acid and/or propionic acid etc. Preferred inorganic acids are hydrochloric and sulphuric acids. It is also possible to use the acids which have already formed an acid addition salt with one of the active substances. Of the organic acids, ascorbic acid, fumaric acid and citric acid are preferred. If desired, mixtures of the above acids may be used, particularly in the case of acids which have other properties in addition to their acidifying qualities, e.g. as flavourings, antioxidants or complexing agents, such as citric acid or ascorbic acid, for example. According to the invention, it is particularly preferred to use hydrochloric acid to adjust the pH.

According to the invention, the addition of editic acid (EDTA) or one of the known salts thereof, sodium edetate, as stabiliser or complexing agent is unnecessary in the present formulation. Other embodiments may contain this compound or these compounds. In a preferred embodiment the content based on sodium edetate is less than 100mg/100ml, preferably less than 50mg/100 ml, more preferably less than 20mg/100 ml. Generally, inhalable solutions in which the content of sodium edetate is from 0 to 10mg/100ml are preferred.

Co-solvents and/or other excipients may be added to the propellant-free inhalable solutions according to the invention. Preferred co-solvents are those which contain hydroxyl groups or other polar groups, e.g. alcohols - particularly isopropyl alcohol, glycols - particularly propyleneglycol, polyethyleneglycol, polypropyleneglycol, glycolether, glycerol, polyoxyethylene alcohols and polyoxyethylene fatty acid esters. The terms excipients and additives in this context denote any pharmacologically acceptable substance which is not an active substance but which can be formulated with the active substance or substances in the pharmacologically suitable solvent in order to improve the qualitative properties of the active substance formulation. These substances may have a pharmacological effect or not, but at least no undesirable pharmacological effect. The excipients and additives include, for example, surfactants such as soya lecithin, oleic acid, sorbitan esters, such as polysorbates, polyvinylpyrrolidone, other stabilisers, complexing agents, antioxidants and/or preservatives which guarantee or prolong the shelf life of the finished pharmaceutical formulation, flavourings, vitamins and/or other additives known in the art. The additives also include pharmacologically acceptable salts such as sodium chloride as isotonic agents.

The preferred excipients include antioxidants such as ascorbic acid, for example, provided that it has not already been used to adjust the pH, vitamin A, vitamin E, tocopherols and similar vitamins and provitamins occurring in the human body.

Preservatives may be used to protect the formulation from contamination with pathogens. Suitable preservatives are those which are known in the art, particularly cetyl pyridinium chloride, benzalkonium chloride or benzoic acid or benzoates such as sodium benzoate in the concentration known from the prior art. The preservatives mentioned above are preferably present in concentrations of up to 50mg/100ml, more preferably between 5 and 20mg/100ml.

Preferred formulations contain, in addition to the solvent water and the combination of active substances **1** and **2**, only benzalkonium chloride and sodium edetate. In another preferred embodiment, no sodium edetate is present.

The propellant-free inhalable solutions according to the invention are administered in particular using inhalers of the kind which are capable of nebulising a small amount of a liquid formulation in the therapeutic dose within a few seconds to produce an aerosol suitable for therapeutic inhalation.

Within the scope of the present invention, preferred inhalers are those in which a quantity of less than 100µL, preferably less than 50µL, more preferably between 10 and 30µL of active substance solution can be nebulised in preferably one spray action to form an aerosol with an average particle size of less than 20µm, preferably less than 10µm, in such a way that the inhalable part of the aerosol corresponds to the therapeutically effective quantity.

An apparatus of this kind for propellant-free delivery of a metered quantity of a liquid pharmaceutical composition for inhalation is described for example in International Patent Application WO 91/14468 and also in WO 97/12687 (cf. in particular Figures 6a and 6b). The nebulisers (devices) described therein are known by the name Respimat®.

This nebuliser (Respimat®) can advantageously be used to produce the inhalable aerosols according to the invention containing the combination of active substances **1** and **2**. Because of its cylindrical shape and handy size of less than 9 to 15 cm long and 2 to 4 cm wide, this device can be carried at all times by the patient. The nebuliser sprays a defined volume of pharmaceutical formulation using high pressures through small nozzles so as to produce inhalable aerosols.

The preferred atomiser essentially consists of an upper housing part, a pump housing, a nozzle, a locking mechanism, a spring housing, a spring and a storage container, characterised by
- a pump housing which is secured in the upper housing part and which comprises at one end a nozzle body with the nozzle or nozzle arrangement,
- a hollow plunger with valve body,
- a power takeoff flange in which the hollow plunger is secured and which is located in the upper housing part,
- a locking mechanism situated in the upper housing part,
- a spring housing with the spring contained therein, which is rotatably mounted on the upper housing part by means of a rotary bearing,
- a lower housing part which is fitted onto the spring housing in the axial direction.

The hollow plunger with valve body corresponds to a device disclosed in WO97/12687. It projects partially into the cylinder of the pump housing and is axially movable within the cylinder. Reference is made in particular to Figures 1 to 4, especially Figure 3, and the relevant parts of the description. The hollow plunger with valve body exerts a pressure of 5 to 60 Mpa (about 50 to 600 bar), preferably 10 to 60 Mpa (about 100 to 600 bar) on the fluid, the measured amount of active substance solution, at its high pressure end at the moment when the spring is actuated. Volumes of 10 to 50 microlitres are preferred, while volumes of 10 to 20 microlitres are particularly preferred and a volume of 15 microlitres per spray is most particularly preferred.

The valve body is preferably mounted at the end of the hollow plunger facing the valve body.

The nozzle in the nozzle body is preferably microstructured, i.e. produced by microtechnology. Microstructured nozzle bodies are disclosed for example in WO-94/07607; reference is hereby made to the contents of this specification, particularly Figure 1 therein and the associated description.

The nozzle body consists for example of two sheets of glass and/or silicon firmly joined together, at least one of which has one or more microstructured channels which connect the nozzle inlet end to the nozzle outlet end. At the nozzle outlet end there is at least one round or non-round opening 2 to 10 microns deep and 5 to 15 microns wide, the depth preferably being 4.5 to 6.5 microns while the length is preferably 7 to 9 microns.

In the case of a plurality of nozzle openings, preferably two, the directions of spraying of the nozzles in the nozzle body may extend parallel to one another or may be inclined relative to one another in the direction of the nozzle opening. In a nozzle body with at least two nozzle openings at the outlet end the directions of spraying may be at an angle of 20 to 160° to one another, preferably 60 to 150°, most preferably 80 to 100°. The nozzle openings are preferably arranged at a spacing of 10 to 200 microns, more preferably at a spacing of 10 to 100 microns, most preferably 30 to 70 microns. Spacings of 50 microns are most preferred. The directions of spraying will therefore meet in the vicinity of the nozzle openings.

The liquid pharmaceutical preparation strikes the nozzle body with an entry pressure of up to 600 bar, preferably 200 to 300 bar, and is atomised into an inhalable aerosol through the nozzle openings. The preferred particle or droplet sizes of the aerosol are up to 20 microns, preferably 3 to 10 microns.

The locking mechanism contains a spring, preferably a cylindrical helical compression spring, as a store for the mechanical energy. The spring acts on the power takeoff flange as an actuating member the movement of which is determined by the position of a locking member. The travel of the power takeoff flange is precisely limited by an upper and lower stop. The spring is preferably biased, via a power step-up gear, e.g. a helical thrust gear, by an external torque which is produced when the upper housing part is rotated counter to the spring housing in the lower housing part. In this case, the upper housing part and the power takeoff flange have a single or multiple V-shaped gear.

The locking member with engaging locking surfaces is arranged in a ring around the power takeoff flange. It consists, for example, of a ring of plastic or metal which is inherently radially elastically deformable. The ring is arranged in a plane at right angles to the atomiser axis. After the biasing of the spring, the locking surfaces of the locking member move into the path of the power takeoff flange and prevent the spring from relaxing. The locking member is actuated by means of a button. The actuating button is connected or coupled to the locking member. In order to actuate the locking mechanism, the actuating button is moved parallel to the annular plane, preferably into the atomiser; this causes the deformable ring to deform in the annual plane. Details of the construction of the locking mechanism are given in WO 97/20590.

The lower housing part is pushed axially over the spring housing and covers the mounting, the drive of the spindle and the storage container for the fluid.

When the atomiser is actuated the upper housing part is rotated relative to the lower housing part, the lower housing part taking the spring housing with it. The spring is thereby compressed and biased by means of the helical thrust gear and the locking mechanism engages automatically. The angle of rotation is preferably a whole-number fraction of 360 degrees, e.g. 180 degrees. At the same time as the spring is biased, the power takeoff part in the upper housing part is moved along by a given distance, the hollow plunger is withdrawn inside the cylinder in the pump housing, as a result of which some of the fluid is sucked out of the storage container and into the high pressure chamber in front of the nozzle.

If desired, a number of exchangeable storage containers which contain the fluid to be atomised may be pushed into the atomiser one after another and used in succession. The storage container contains the aqueous aerosol preparation according to the invention.

The atomising process is initiated by pressing gently on the actuating button. As a result, the locking mechanism opens up the path for the power takeoff member. The biased spring pushes the plunger into the cylinder of the pump housing. The fluid leaves the nozzle of the atomiser in atomised form.

Further details of construction are disclosed in PCT Applications WO 97/12683 and WO 97/20590, to which reference is hereby made.

The components of the atomiser (nebuliser) are made of a material which is suitable for its purpose. The housing of the atomiser and, if its operation permits, other parts as well are preferably made of plastics, e.g. by injection moulding. For medicinal purposes, physiologically safe materials are used.

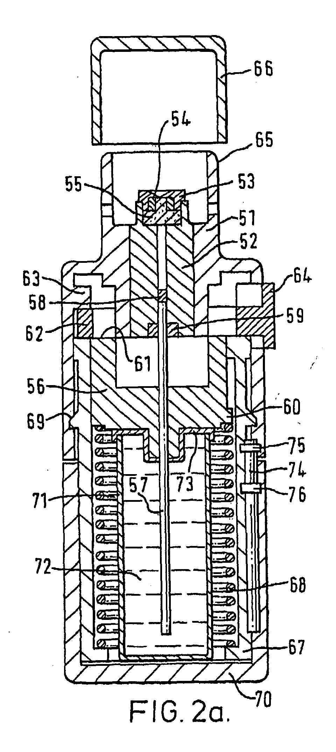

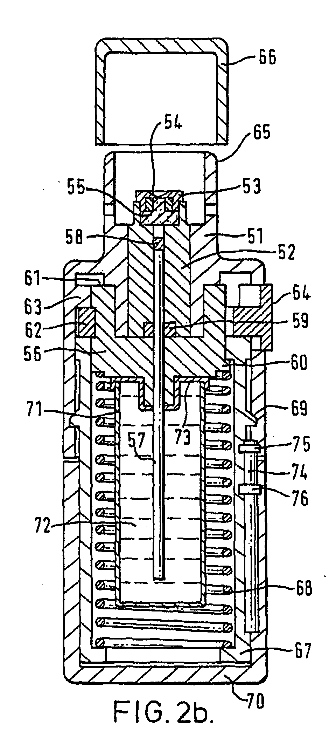

Figures 2a/b attached to this patent application, which are identical to Figures 6a/b of WO 97/12687, show the nebuliser (Respimat®) which can advantageously be used for inhaling the aqueous aerosol preparations according to the invention.

Figure 2a shows a longitudinal section through the atomiser with the spring biased while Figure 2b shows a longitudinal section through the atomiser with the spring relaxed.

The upper housing part (51) contains the pump housing (52) on the end of which is mounted the holder (53) for the atomiser nozzle. In the holder is the nozzle body (54) and a filter (55). The hollow plunger (57) fixed in the power takeoff flange (56) of the locking mechanism projects partially into the cylinder of the pump housing. At its end the hollow plunger carries the valve body (58). The hollow plunger is sealed off by means of the seal (59). Inside the upper housing part is the stop (60) on which the power takeoff flange abuts when the spring is relaxed. On the power takeoff flange is the stop (61) on which the power takeoff flange abuts when the spring is biased. After the biasing of the spring the locking member (62) moves between the stop (61) and a support (63) in the upper housing part. The actuating button (64) is connected to the locking member. The upper housing part ends in the mouthpiece (65) and is sealed off by means of the protective cover (66) which can be placed thereon.

The spring housing (67) with compression spring (68) is rotatably mounted on the upper housing part by means of the snap-in lugs (69) and rotary bearing. The lower housing part (70) is pushed over the spring housing. Inside the spring housing is the exchangeable storage container (71) for the fluid (72) which is to be atomised. The storage container is sealed off by the stopper (73) through which the hollow plunger projects into the storage container and is immersed at its end in the fluid (supply of active substance solution).

The spindle (74) for the mechanical counter is mounted in the covering of the spring housing. At the end of the spindle facing the upper housing part is the drive pinion (75). The slider (76) sits on the spindle.

The nebuliser described above is suitable for nebulising the aerosol preparations according to the invention to produce an aerosol suitable for inhalation..

If the formulation according to the invention is nebulised using the method described above (Respimat®) the quantity delivered should correspond to a defined quantity with a tolerance of not more than 25%, preferably 20% of this amount in at least 97%, preferably at least 98% of all operations of the inhaler (spray actuations). Preferably, between 5 and 30 mg of formulation, most preferably between 5 and 20 mg of formulation are delivered as a defined mass on each actuation.

However, the formulation according to the invention may also be nebulised by means of inhalers other than those described above, e.g. jet stream inhalers.

Accordingly, in a further aspect, the invention relates to pharmaceutical formulations in the form of propellant-free inhalable solutions or suspensions as described above combined with a device suitable for administering these formulations, preferably in conjunction with the Respimat®. Preferably, the invention relates to propellant-free inhalable solutions or suspensions characterised by the combination of active substances **1** and **2** according to the invention in conjunction with the device known by the name Respimat®. In addition, the present invention relates to the above-mentioned devices for inhalation, preferably the Respimat®, characterised in that they contain the propellant-free inhalable solutions or suspensions according to the invention as described hereinbefore.

The propellant-free inhalable solutions or suspensions according to the invention may take the form of concentrates or sterile inhalable solutions or suspensions ready for use, as well as the above-mentioned solutions and suspensions designed for use in a Respimat®. Formulations ready for use may be produced from the concentrates, for example, by the addition of isotonic saline solutions. Sterile formulations ready for use may be administered using energy-operated fixed or portable nebulisers which produce inhalable aerosols by means of ultrasound or compressed air by the Venturi principle or other principles.

Accordingly, in another aspect, the present invention relates to pharmaceutical compositions in the form of propellant-free inhalable solutions or suspensions as described hereinbefore which take the form of concentrates or sterile formulations ready for use, combined with a device suitable for administering these solutions, characterised in that the device is an energy-operated free-standing or portable nebuliser which produces inhalable aerosols by means of ultrasound or compressed air by the Venturi principle or other methods.

The Examples which follow serve to illustrate the present invention in more detail without restricting the scope of the invention to the following embodiments by way of example.

### Preparation of the compounds of the formula 1:

The synthesis of compounds of the formula **1**, especially of the compound of the formula **1a** and of the formula **1b** is described in the prior art, in particular in WO 02/30928, WO 03/000265 and in WO 02/32899.

The synthesis of the compound **2** is described e.g. in EP 0 771 794 A1, in particular as example 140 on page 109 therein.

### Test on the biological activity

In diseases such as COPD and asthma neural pathways are believed to play an important role. These pathways can be classified under cholinergic pathways, in which acetylcholine is the end-transmitter, and tachykininergic pathways in which neuropeptides such as substance P or neurokinin A serve as end-transmitters. The example presented refers to the ability of these neural mechanisms to influence bronchospasm, but neural mechanisms can also be involved in regulation of mucus secretion or inflammatory processes, and the synergistic effects of compounds of formula **1** and formula **2** should not be considered to be restricted to effects on bronchospasm.

This example describes the effects of a compound of the formula **1** in combination with a compound of the formula **2** on the contraction of bronchus. As an example of the compound of the formula **1** tiotropium bromide was chosen, but this test may be performed with any compound of the formula **1**, in particular with a compound of the formula **1b**, achieving comparable results. Male Hartley guinea pigs (300-700g) were killed with CO₂ asphyxiation. The main bronchial rings were excised. The individual rings were opened by cutting through the cartilage and suspended vertically between parallel platinum wire field electrodes in the organ baths containing Krebs Henseleit (KH) solution (composition mmol/L: NaCl 119, KCl 4.7, MgSO₄ 1.2, CaCl₂ 2.5, KH₂PO₄ 1.2, NaHCO₃ 25 and glucose 11.7) at 37°C and gassed with O₂/CO₂ (95:5, v/v). The preparations were allowed to equilibrate for 1 h with frequent washing. All experiments were performed in the presence of indomethacin (5 µmol/L) to prevent the formation of contractile prostaglandins. Contractions were measured by an isometric force transducer (TB-611T, Nihon Kohden, Tokyo, Japan) under a resting tension of 0.3 g. The preparation in this manner were contracted by electrical field stimulation (EFS: 30 V, 0.5 ms duration and a frequency of 8 Hz for 15 s). The EFS induced reproducible biphasic contraction. The fast phase contraction was mediated by acetylcholine (cholinergic phase). The slow phase contraction was mediated by tachykinins such as substance P and neurokinin A (tachykininergic phase) (Br. J. Pharmacol. 1996, 117 (5), 967-973). After the first EFS-induced contractions, the bronchi were treated with 10 nmol/L tiotropium bromide and/or 30 µmol/L compound **2**. The concentrations refer to the final bath concentrations. EFS-induced contractions were measured 20 min after the additions of the compounds. Tiotropium bromide inhibited the cholinergic contraction. Compound **2** inhibited the tachykininergic contraction. The combination of tiotropium bromide and compound **2** could inhibit both the cholinergic and tachykininergic contractions. The combination of the compounds inhibited the cholinergic contraction more strongly than tiotropium bromide itself. The combination of the compounds inhibited the tachykininergic contraction more potently than compound **2** itself:

The inhibitions of both cholinergic response and tachykininergic response in the pulmonary system are effective in airway diseases such as asthma and COPD (Curr. Opin. Chem. Biol. 2000, 4 (4) 412-419, Eur. J. Pharmacol. 2001, 429 (1-3), 239-250). Therefore, the combination of active substances according to this invention is particularly suitable for treating inflammatory and/or obstructive diseases of the respiratory tract, particularly asthma or chronic obstructive pulmonary disease (COPD), and complications thereof such as pulmonary hypertension, as well as allergic and non-allergic rhinitis.

**Table 1.**

| Effects of compounds on the EFS-induced cholinergic and tachykininergic contraction in the guinea pig main bronchus. The EFS-induced contractions were measured in the absence (Vehicle) or presence of 10 nmol/L tiotropium bromide (Tiotropium bromide), 30 *µ*mol/L Compound **2** (Compound **2)** or 10 nmol/L tiotropium bromide and 30 *µ*mol/l Compound **2** (Combination). Dimethyl sulfoxide (0.1 %) and distilled water (0.1 %) were added as vehicles. n is the number of trials. | | | |
|---|---|---|---|
| Compounds | n | % of the first contraction | |
| | | Cholinergic Contraction | Tachykininergic contraction |
| Vehicle | 5 | 107.7 ± 2.8 | 98.7 ± 4.5 |
| Tiotropium bromide | 5 | 59.7 ± 6.3** | 90.9 ± 8.3 |
| Compound **2** | 5 | 105.8 ± 2.4 | 66.1 ± 6.5 ** |
| Combination | 5 | 43.2 ± 3.5*** | 54.7 ± 10.3** |

| | | | |
|---|---|---|---|
| Values are means ± S.E.M (standard error of the mean) **, *** p < 0.01, 0.0001 vs the vehicle groups (Student's t-test). | | | |

### Examples of Formulations

### A) Inhalable powders:

1)

| **Ingredients** | **µg per capsule** |
|---|---|
| tiotropium bromide | 21.7 |
| compound **2** | 250 |
| lactose | 4728.3 |
| total | 5000 |

2)

| **Ingredients** | ***µ*g per capsule** |
|---|---|
| tiotropium bromide | 8.5 |
| compound **2** | 500 |
| lactose | 4491.5 |
| total | 5000 |

3)

| **Ingredients** | ***µ*g per capsule** |
|---|---|
| tiotropium bromide | 14.5 |
| compound **2** | 900 |
| lactose | 4085.5 |
| total | 5000 |

4)

| **Ingredients** | **µg per capsule** |
|---|---|
| tiotropium bromide | 17.5 |
| compound **2** | 1700 |
| lactose | 3282.5 |
| total | 5000 |

5)

| **Ingredients** | ***µ*g per capsule** |
|---|---|
| tiotropium bromide | 24.5 |
| compound **2** | 2300 |
| lactose | 2675.5 |
| total | 5000 |

6)

| **Ingredients** | **µg per capsule** |
|---|---|
| tiotropium bromide | 19 |
| compound **2** | 3000 |
| lactose | 1981 |
| total | 5000 |

7)

| **Ingredients** | **µg per capsule** |
|---|---|
| tiotropium bromide | 20.5 |
| compound **2** | 3750 |
| lactose | 2229.5 |
| total | 6000 |

8)

| **Ingredients** | **µg per capsule** |
|---|---|
| tiotropium bromide x H₂O | 22.5 |
| compound **2** | 1150 |
| lactose | 3827.5 |
| total | 5000 |

9)

| **Ingredients** | **µg per capsule** |
|---|---|
| tiotropium bromide x H₂O | 19.0 |
| compound **2** | 1350 |
| lactose | 3631 |
| total | 5000 |

10)

| **Ingredients** | **µg per capsule** |
|---|---|
| compound **1b** (as bromide) | 35 |
| compound **2** | 900 |
| lactose | 4065 |
| **Total** | 5000 |

11)

| **Ingredients** | **µg per capsule** |
|---|---|
| compound **1b** (as bromide) | 75 |
| compound **2** | 250 |
| lactose | 4675 |
| **Total** | 5000 |

12)

| **Ingredients** | **µg per capsule** |
|---|---|
| compound **1b** (as bromide) | 160 |
| compound **2** | 650 |
| lactose | 4190 |
| **Total** | 5000 |

13)

| **Ingredients** | **µg per capsule** |
|---|---|
| compound **1b** (as bromide) | 265 |
| compound **2** | 1350 |
| lactose | 3385 |
| **Total** | 5000 |

14)

| **ingredients** | **µg per capsule** |
|---|---|
| compound **1b** (as bromide) | 375 |
| compound **2** | 2350 |
| lactose | 2275 |
| **Total** | 5000 |

### B) Propellant-containing aerosols for inhalation:

1) Suspension aerosol:

| **Ingredients** | **% by weight** |
|---|---|
| tiotropium bromide | 0.003 |
| compound **2** | 0.030 |
| soya lecithin | 0.2 |
| TG 134a TG227 = 2:3 | ad 100 |

2) Suspension aerosol:

| **Ingredients** | **% by weight** |
|---|---|
| tiotropium bromide | 0.0029 |
| compound **2** | 0.040 |
| absolute ethanol | 0.5 |
| isopropyl myristate | 0.1 |
| TG 227 | ad 100 |

3) Suspension aerosol:

| **Ingredients** | **% by weight** |
|---|---|
| tiotropium bromide | 0.0029 |
| compound **2** | 0.095 |
| absolute ethanol | 0.5 |
| isopropyl myristate | 0.1 |
| TG 227 | ad 100 |

4) Suspension aerosol:

| **Ingredients** | **% by weight** |
|---|---|
| tiotropium bromide | 0.029 |
| compound **2** | 0.250 |
| absolute ethanol | 0.5 |
| isopropyl myristate | 0.1 |
| TG 227 | ad 100 |

5)

| **Ingredients** | **% by weight** |
|---|---|
| **1b** (as bromide) | 0.020 |
| compound **2** | 0.066 |
| soya lecithin | 0.2 |
| TG 134a: TG 227 = 2:3 | ad 100 |

6)

| **Ingredients** | **% by weight** |
|---|---|
| **1b** (as bromide) | 0.0045 |
| compound **2** | 0.0550 |
| absolute ethanol | 0.5 |
| isopropyl myristate | 0.1 |
| TG 227 | ad 100 |

## Claims

1. Pharmaceutical composition, **characterised in that** it contains an anticholinergic of the formula (**1**) 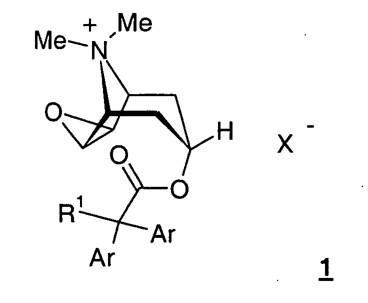 wherein
X⁻ represents chlorine, bromine, iodine, methanesulphonate or trifluoromethanesulphonate;
R¹ represents hydroxy or methyl;
Ar represents phenyl or thienyl;
in combination with the compound of the formula (**2**) 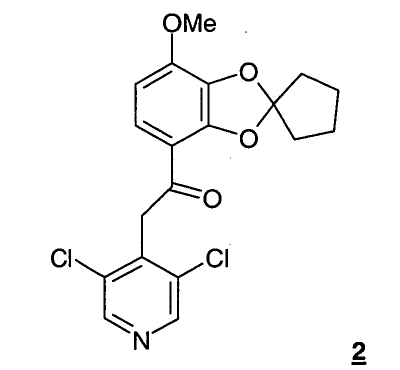 optionally in the form of a pharmacologically acceptable acid addition salt thereof, optionally in the form of a solvate or hydrate and optionally together with a pharmaceutically acceptable excipient.

2. Pharmaceutical composition according to claim 1, **characterised in that 1** is a compound of the formula (**1a**) 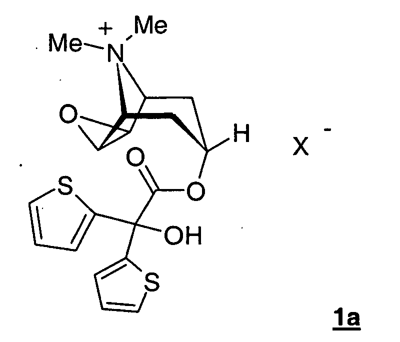 wherein
X⁻ represents chlorine, bromine, iodine, methanesulphonate or trifluoromethanesulphonate,
optionally in the form of a pharmacologically acceptable acid addition salt thereof, optionally in the form of a solvate or hydrate and optionally together with a pharmaceutically acceptable excipient.

3. Pharmaceutical composition according to claim 2, **characterised in that** X represents bromine.

4. Pharmaceutical composition according to claim 1, **characterised in that** **1** is a compound of the formula (**1b**) 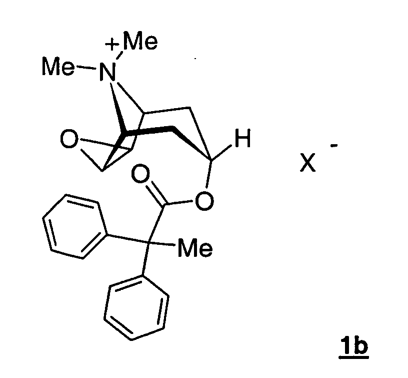 wherein
X⁻ represents chlorine, bromine, iodine, methanesulphonate or trifluoromethanesulphonate,
optionally in the form of a pharmacologically acceptable acid addition salt thereof, optionally in the form of a solvate or hydrate and optionally together with a pharmaceutically acceptable excipient.

5. Pharmaceutical composition according to claim 4, **characterised in that** X represents bromine.

6. Pharmaceutical composition according to one of claims 1 to 5, **characterised in that** the active substances **1** and **2** are present either together in a single formulation or in two separate formulations.

7. Pharmaceutical composition according to one of claims 1 to 6, **characterised in that** the weight ratios of **1** to **2** are in the range from 1:4000 to 8:1, preferably from 1:1000 to 1:1.2.

8. Pharmaceutical composition according to one of claims 2 to 6, **characterised in that** the weight ratios of **1a** to **2** are in the range from 1:4000 to 1:2:5, preferably from 1:1000 to 1:12.5.

9. Pharmaceutical composition according to one of claims 4 to 6, **characterised in that** the weight ratios of **1b** to **2** are in the range from 1:4000 to 8:1, preferably from 1:1000 to 1:1.2.

10. Pharmaceutical composition according to one of claims 1 to 9, **characterised in that** the total dosage per single dose of the active substance combination **1** and **2** is in the range of 25 to 10000µg, preferably from 100 to 5800µg.

11. Pharmaceutical composition according to one of claims 1 to 10, **characterised in that** it is in the form of a formulation suitable for inhalation.

12. Pharmaceutical composition according to claim 11, **characterised in that** it is a formulation selected from among inhalable powders, propellant-containing metering aerosols and propellant-free inhalable solutions or suspensions.

13. Pharmaceutical composition according to claim 12, **characterised in that** it is an inhalable powder which contains **1** and **2** in admixture with suitable physiologically acceptable excipients selected from among the monosaccharides, disaccharides, oligo- and polysaccharides, cyclodextrines, polyalcohols, salts, or mixtures of these excipients with one another.

14. Inhalable powder according to claim 13, **characterised in that** the excipient has a maximum average particle size of up to 250µm, preferably between 10 and 150µm.

15. Pharmaceutical composition according to claim 12, **characterised in that** it is an inhalable powder which contains only the active substances **1** and **2** as its ingredients.

16. Pharmaceutical composition according to claim 12, **characterised in that** it is a propellant-containing inhalable aerosol which contains **1** and **2** in dissolved or dispersed form.

17. Pharmaceutical composition in the form of a propellant-containing inhalable aerosol according to claim 16, **characterised in that** it contains, as propellant gas, hydrocarbons such as n-propane, n-butane or isobutane or halohydrocarbons such as chlorinated and/or fluorinated derivatives of methane, ethane, propane, butane, cyclopropane or cyclobutane.

18. Pharmaceutical composition in the form of a propellant-containing inhalable aerosol according to claim 17, **characterised in that** the propellant gas is TG11, TG12, TG134a ( 1,1,1,2-tetrafluoroethane), TG227 (1,1,1,2,3,3,3-heptafluoropropane) or a mixture thereof.

19. Pharmaceutical composition according to claim 12, **characterised in that** it is a propellant-free inhalable solution or suspension which contains water, ethanol or a mixture of water and ethanol as solvent.

20. Pharmaceutical composition in the form of an inhalable solution or suspension according to claim 19, **characterised in that** the pH is 2 to 7, preferably 2 to 5.

21. Capsules, **characterised in that** they contain an inhalable powder according to claim 13 or 14.

22. Use of a capsule according to claim 15 in an inhaler, preferably in a Handyhaler.

23. Use of an inhalable solution according to one of claims 19 or 20 for nebulising in an inhaler, preferably according to WO 91/14468 or an inhaler as described according to the Figures 6a and 6b of WO 97/12687.

24. Use of a composition according to one of claims 1 to 20 for preparing a medicament for treating pulmonary diseases, in particular inflammatory or obstructive diseases of the respiratory tract.

25. A method of prophylaxis of, treating of, or reducing the exacerbations associated with pulmonary diseases by administering to a patient in need thereof an effective amount of a pharmaceutical composition according to one or more of the claims 1 to 20 either in a single combined form, separately, or separately and sequentially where the sequential administration is close in time, or remote in time.

26. The method according to claim 25 wherein the pulmonary disease is asthma, COPD, or another obstructive airways disease exacerbated by bronchial hyperreactivity and bronchospasm.

27. The method according to claim 25 or 26 wherein said administration by inhalation comprises simultaneous or sequential delivery of said combination of therapeutic agents, comprising a compound of the formula **1** and a compound of the formula **2**, in the form of an aerosol or dry powder dispersion.

28. The method according to one or more of the claims 25 to 27, wherein the compound of the formula **1** is a compound of the formula **1a**.

29. The method according to one or more of the claims 25 to 27, wherein the compound of the formula **1** is a compound of the formula **1b.**

30. A package comprising a pharmaceutical composition according to one or more of the claims 1 to 22 for insertion into a device of simultaneous or sequential delivery of said pharmaceutical composition in the form of an aerosol or dry powder dispersion, to a mammal in need of treatment.

31. Inhaler comprising a pharmaceutical composition according to one or more of the claims 1 to 22 for simultaneous or sequential delivery of said pharmaceutical composition in the form of an aerosol or dry powder dispersion, to a mammal in need of treatment.
